# EUROPEAN PATENT SPECIFICATION

(11) **EP 4 511 094 B1**
(45) Date of publication and mention of the grant of the patent: **11.11.2026**
(21) Application number: 23792384.2
(22) Date of filing: 17.04.2023
(51) Int. Cl.: A61M 16/06, A61B 5/00

(54) **LINER FOR USE WITH RESPIRATORY MASK**
AUSKLEIDUNG ZUR VERWENDUNG MIT EINER ATEMMASKE
DOUBLURE DESTINÉE À ÊTRE UTILISÉE AVEC UN MASQUE RESPIRATOIRE

(30) Priority: 18.04.2022 US 202217723290
(43) Date of publication of application: 26.02.2025
(73) Proprietor: Naturs Design, Inc., Hillsdale, MI 49242 (US)
(72) Inventor: RUTAN, Robert M., Sandpoint, Idaho 83864 (US); RUTAN, Nathan, Lakewood, Colorado 80228 (US)
(74) Representative: Kilburn & Strode LLP
(86) International application number: PCT/US2023/018847
(87) International publication number: WO 2023/205089

(56) References cited:
- WO-A1-01/43804
- WO-A1-2008/077242
- CN-U- 211 132 571
- CN-U- 212 997 866
- CN-U- 214 340 294
- US-A1- 2009 030 289
- US-A1- 2015 352 307
- US-A1- 2016 175 552
- US-A1- 2016 279 359
- US-A1- 2019 001 093
- US-A1- 2021 236 057
- US-A1- 2022 241 535

## Description

### CROSS-REFERENCE TO RELATED APPLICATIONS

This application claims priority to U.S. Application No. 17/723,290 filed April 18, 2022.

### TECHNICAL FIELD

Embodiments relate to a liner for use with a respiratory mask, such as a CPAP mask.

### BACKGROUND

Obstructive sleep apnea is a serious and potentially fatal medical condition in which a person's airway becomes physically blocked multiple times during sleep, restricting oxygen intake and causing the person to awake gasping for breath. Possible effects of the condition include extreme fatigue, high blood pressure, strokes, heart attacks, and sometimes even death.

One of the most common treatments of obstructive sleep apnea is the use of a continuous positive airway pressure (CPAP) machine. These machines deliver a continuous flow of pressurized air to the airway through a hose and mask fitted to the face. Patient compliance is a major problem with CPAP users, however, due to discomfort, air leaks, and general ineffectiveness. It is estimated that up to 50% of users discontinue use.

Most CPAP masks currently available are made from silicone, rubber, vinyl, or a nylon-based fabric. These materials are typically water and gas impermeable, which can block off pores, cause sweating, and create pressure marks on the face, increasing the discomfort of the mask. Furthermore, most mask manufacturers recommend against the use of skin or face cream with CPAP masks since the mask material directly contacts the skin. This is a problem for many users, especially those that have dry skin and depend on night cream for skin care.
US 2019/001093 A1 to Naturs Design, Inc. discloses a liner for use with a respiratory mask with a frame portion that is detachable from a cushion portion. US 2015/352307 A1 to Naturs Design, Inc. describes a liner for use with a respiratory mask having a face-engaging portion and nasal pillows. US 2016/279359 A1 to Taichung Veterans General Hospital discloses an inductive breathing mask and a respiratory care system which may detect the compression level of the user's face. CN 212997866 U to The First Affiliated Hospital of Guangzhou Medical University describes a face mask with real-time blood oxygen display. CN 211132571 U to Shenzhen EEGSmart Technology Co., Ltd discloses a respiratory mask and breathing device. WO 01/43804 A1 to Compumedics Limited relates to a breathing mask for use in monitoring a patient. WO 2008/077242 A1 to Excel Tech Ltd discloses a method, system and device for sleep stage determination using electrodes.

### SUMMARY OF THE INVENTION

The present invention provides a liner for use with a respiratory mask having a face-engaging portion as defined in claim 1. The present invention also provides a liner system for use with a respiratory mask having a face-engaging portion and a forehead stabilizer portion as defined in claim 6. Further preferred embodiments of the invention are defined in the dependent claims.

### BRIEF DESCRIPTION OF THE DRAWINGS

FIGURE 1 is a top plan view of a liner according to an embodiment, such as for use with a full-face respiratory mask;
FIGURE 2 is a top plan view of a liner according to an embodiment, such as for use with a nasal respiratory mask;
FIGURE 3 is a top plan view of a liner according to an embodiment, such as for use with a partial-face respiratory mask;
FIGURE 4 is a top plan view of a forehead liner according to an embodiment;
FIGURE 5 is a perspective view of an exemplary full-face respiratory mask;
FIGURE 6 is an illustration of placement of a liner in accordance with an embodiment on the face-engaging portion of a respiratory mask;
FIGURE 7 is an illustration of a user engaging the liner placed on the mask, fitting her nose and mouth into the liner opening;
FIGURE 8 is an illustration of a user returning her head to an upright position while holding the mask and liner against her face;
FIGURE 9 is an illustration of a user attaching the mask straps;
FIGURE 10 is an illustration of a user adjusting the positioning of the liner by pulling on the second portion of the liner body protruding beyond the perimeter of the face-engaging portion;
FIGURE 11 is a top plan view of a liner according to an embodiment, such as for use with a nasal pillow mask;
FIGURE 12 is a top plan view of a liner according to another embodiment for use with a nasal pillow mask;
FIGURE 13 is a top plan view of a liner according to an embodiment, such as for use with a hybrid nasal pillow/partial face mask;
FIGURE 14 is a photograph of placement of a first pair of apertures of a liner as in FIG. 11 in accordance with an embodiment over the nasal pillows of a nasal pillow mask;
FIGURE 15 is a photograph illustrating a user folding the liner of FIG. 11 so that a second pair of apertures is aligned with the nasal pillows;
FIGURE 16 is a photograph illustrating a user adjusting the positioning of the liner of FIG. 11 by pulling on the portion of the liner body protruding beyond the perimeter of the nasal pillow mask;
FIGURE 17 is a photograph of placement of a first pair of apertures of a liner as in FIG. 13 in accordance with an embodiment over the nasal pillows of a hybrid nasal pillow/partial face mask;
FIGURE 18 is a photograph illustrating a user folding the liner of FIG. 13 so that a second pair of apertures is aligned with the nasal pillows;
FIGURE 19 is a photograph illustrating alignment of the opening of the liner of FIG. 13 with the face-engaging portion of the hybrid mask;
FIGURE 20 is a photograph illustrating a user engaging the liner of FIG. 13 placed on the hybrid mask, fitting his nose to the nasal pillows and mouth into the liner opening;
FIGURE 21 is a top plan view of a liner according to another embodiment for use with a respiratory mask having a detachable frame;
FIGURE 22 is a top plan of the liner of FIG. 21 annotated with exemplary, non-limiting dimensions for construction;
FIGURE 23 is a photograph of an exemplary respiratory mask having a detachable frame;
FIGURE 24 is a photograph of the liner of FIG. 21 placed on the mask of FIG. 23 with the frame detached;
FIGURE 25 is a photograph of the liner of FIG. 21 placed on the mask of FIG. 23 with the frame attached;
FIGURE 26 is a top plan view of a liner according to an embodiment, such as for use with a hybrid nasal pillow/partial face mask;
FIGURE 27 is a top plan view of a liner with flaps, wherein the liner is annotated with exemplary, non-limiting dimensions for construction;
FIGURE 28 is a photograph of a liner according to FIG. 27 positioned on a respiratory mask and tethered with a strap;
FIGURE 29 is a side view of a one end of a liner according to FIG. 27 tethered to a respiratory mask;
FIGURE 30 is a side view of another end of a liner according to FIG. 27 tethered to a respiratory mask;
FIGURE 31 is a top plan view of another liner with flaps, such as for pediatric use, wherein the liner is annotated with exemplary, non-limiting dimensions for construction;
FIGURE 32 is a photograph of a liner according to FIG. 31 positioned on a respiratory mask and tethered with a strap;
FIGURE 33 is a side view of a one end of a liner according to FIG. 31 tethered to a respiratory mask;
FIGURE 34 is a side view of another end of a liner according to FIG. 31 tethered to a respiratory mask;
FIGURE 35 is a top plan view of a liner with one or more integrated sensors according to an embodiment; and
FIGURE 36 is a top plan view of a forehead liner with one or more integrated sensors according to an embodiment.

### DETAILED DESCRIPTION

As required, detailed embodiments of the present technology are disclosed herein; however, it is to be understood that the disclosed embodiments are merely exemplary of the technology that may be embodied in various and alternative forms. The figures are not necessarily to scale; some features may be exaggerated or minimized to show details of particular components. Therefore, specific structural and functional details disclosed herein are not to be interpreted as limiting, but merely as a representative basis for teaching one skilled in the art to variously employ the present technology.

One or more embodiments disclosed herein provide an accessory capable of improving the comfort, effectiveness, and/or patient compliance of CPAP and other respiratory masks.

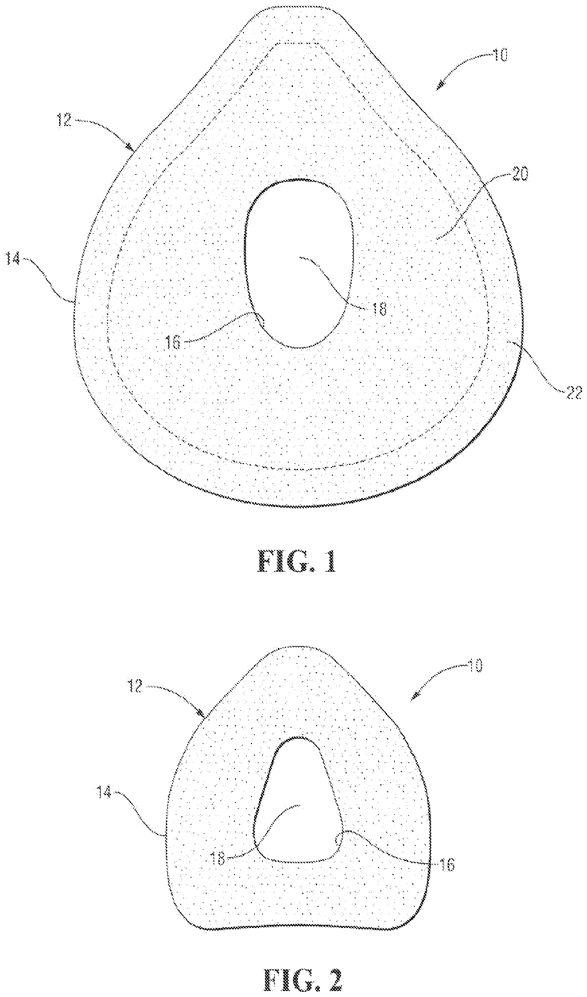

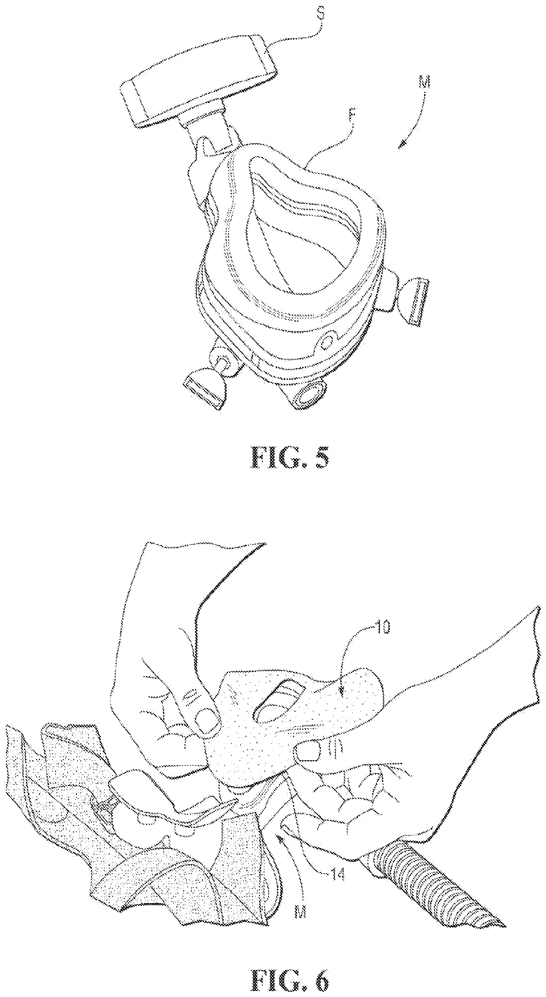

With reference to FIGS. 1-3, a liner for use with a respiratory mask, such as a CPAP mask M (see FIG. 5), is illustrated and designated generally by reference numeral 10. In use, the liner 10 may be positioned between and held in place by the respiratory mask and the face of a user in order to absorb moisture, maintain proper positioning of the mask M, and greatly reduce or eliminate air from leaking between the mask M and the user's face. Although the liner 10 is described herein primarily in relation to use with a CPAP mask, it is understood that the liner 10 may also be used with other types of respiratory masks such as, but not limited to, oxygen masks, respirators, and filtering masks.

In one embodiment, the liner 10 includes a body 12 having an outer edge 14, an inner edge 16, and an opening 18 bounded by the inner edge 16. The body 12 may be generally oval-shaped, elliptical, round, or triangular, or have any other shape appropriate for use with a respiratory mask and is not limited to those shapes depicted herein. The opening 18 is configured to at least partially receive the nose, mouth, or both nose and mouth, depending upon the type of mask, allowing air flow from an air source to be received by the user through the mask M. The opening 18 may be generally elliptical or oval-shaped as shown but is not intended to be limited to these shapes.

Referring to FIGS. 1 and 5, the outer edge 14 of liner 10 may have a shape scaled to a general shape of a face-engaging portion F of the respiratory mask M. As best shown in FIGS. 6-10, a perimeter of the liner outer edge 14, which may be continuous or discontinuous, is larger than a perimeter of the face-engaging portion F, wherein the liner 10 is configured to be releasably held between the mask M and a user's face such that the outer edge 14 extends beyond the mask face-engaging portion F around at least part of its perimeter. As such, as illustrated in an exemplary manner in FIG. 1, the body 12 has a first portion 20 inward of the perimeter of the face-engaging portion F and a second, extending portion 22 outward of the perimeter of the face-engaging portion F. The extending portion 22 extends outwardly from the face-engaging portion F and may generally follow the contours of the user's face. Thus, when the liner 10 is releasably held by the mask M and the user's face, the outer edge 14 is spaced apart from the mask M.

In one embodiment, the outer edge 14 may extend beyond the perimeter of the mask face-engaging portion F by between about 0.25 to 1.0 inches, or more particularly may extend between about 0.5 and 0.75 inches. In general, the area of the extending portion 22 may comprise at least about 5%, 10%, or 15% of the area of the body 12, but larger proportions of area represented by the extending portion 22 are also contemplated. By allowing the outer edge 14 of the liner 10 to loosely protrude beyond the mask M, the extending portion 22 is configured to be in non-adhering communication with a user's face and serves to reduce air leaks from the perimeter of the mask M by acting as a baffle to regulate, limit, or diffuse air flow between the mask M and the skin, thus also stopping any resulting squealing-type noises created by such air leaks.

According to an embodiment, the body 12 is constructed from a single layer of absorbent material, wherein the thickness of the body 12 may be between about 0.005 to 0.05 inches, although these dimensions are not intended to be limiting. In one embodiment, the material may include cotton. In another embodiment, the material may include another material, such as silicone, with cotton embedded therein. However, it is understood that any material with suitable absorption and comfort properties may be used. In further accordance with an embodiment, the material used for the construction of the body 12 may be stretchable to aid in adjusting and customizing the fit of the liner 10 to a particular user as described below. The absorbent material may function to absorb moisture and/or oils from the user's skin and enable the mask M to maintain a consistent and comfortable position with respect to the user's face when in use.

In a CPAP system, an air source (not shown) delivers a constant flow of pressurized and humidified air to the CPAP mask M. Due to the moisture of the humidified air, facial perspiration (such as due to contact with the mask material), and oil from the skin, the mask M may slip on the user's face, thus leaking air and awakening the user during sleep. The liner 10 may absorb such moisture and wick it away from the face and mask surfaces. As a result, proper positioning of the mask M with respect to the skin may be maintained, thus eliminating or greatly reducing air leaks and facilitating the ability for a user to wear their CPAP mask successfully throughout the night.

The single layer construction of the liner 10 may act as a sort of "second skin" upon the user's face. As such, the liner 10 is able to provide its baffle function without detracting from the prescribed fit of the mask M since the liner 10 does not appreciably alter the distance of the face-engaging portion F from the user's face. Pressure markings from the mask M may also be reduced or eliminated by use of the liner 10. Furthermore, the absorbent liner material may make use of facial creams possible while wearing the mask M, since direct contact of the skin with the mask material is avoided.

According to an embodiment, the liner 10 is held in place by the pressure of the respiratory mask M upon the face (e.g., by straps around the head). While it is contemplated that the liner 10 could be at least partially fastened to the mask M, advantageously neither elastic nor another mechanism for securing the liner 10 to the mask M is required, allowing for ease of use and manufacture. The position of the liner 10 can be adjusted if necessary while the mask M is secured, and the liner 10 is easily removable and replaceable when the mask M is removed.

Respiratory masks, more particularly CPAP masks, are offered in various shapes and sizes, including full-face, nasal, child-sized, and partial-face (hybrid) configurations. Full-face masks typically include a wider bottom region for covering the mouth area and a narrower upper region for covering the nasal area. Nasal masks generally cover the nasal area and not the mouth area. Child-sized masks may have a proportionally smaller size. Partial-face (hybrid) masks generally cover the mouth and may include a nasal interface. It is therefore contemplated that the outer edge 14 of liner 10 may have a shape similar to a general shape of the face-engaging portion F for a selected mask M, wherein the shape of the outer edge 14 may represent a scaled version of the general shape of the face-engaging portion F.

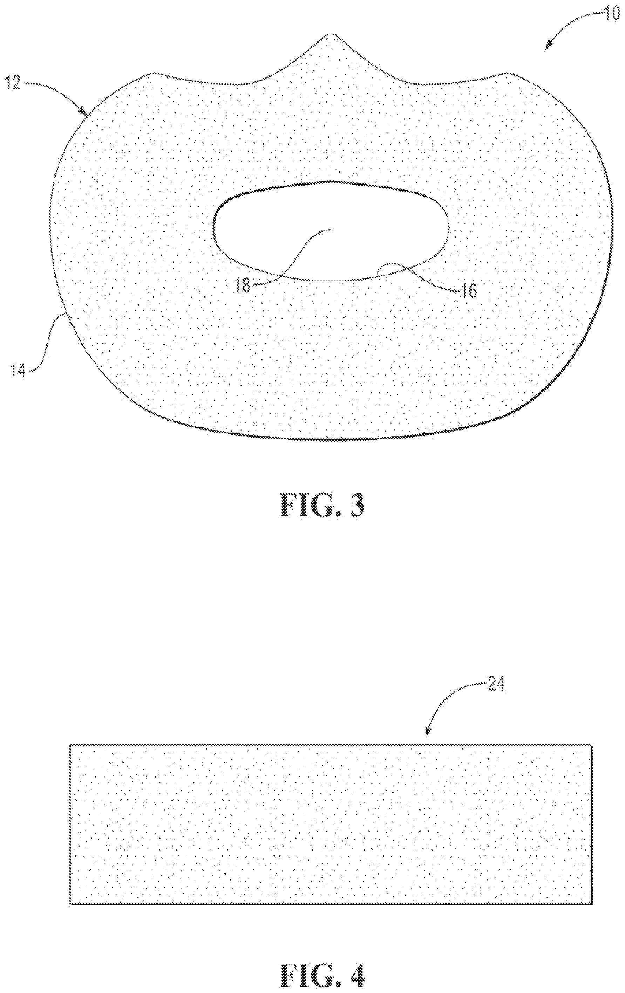

If the liner 10 is to be used with a full-face CPAP mask, the opening 18 may be sized to at least partially receive the user's nose and mouth (see FIG. 1). In this embodiment, the opening 18 may have a length of between about 1.0 to 3.0 inches and a width of between about 1.0 to 1.75 inches, and the body 12 may have a length of between about 4.5 to 7.5 inches and a width of between about 4.5 to 6.5 inches. If the liner 10 is to be used with a nasal CPAP mask, the opening 18 may be sized to at least partially receive the user's nose (see FIG. 2). In this embodiment, the opening 18 may have a length of between about 1.25 to 1.75 inches and a width of between about 0.75 to 1.5 inches, and the body 12 may have a length of between about 3.0 to 4.0 inches and a width of between about 3.0 to 5.0 inches. If the liner 10 is to be used with a partial-face CPAP mask, the opening 18 may be sized to at least partially receive the user's mouth (see FIG. 3). In this embodiment, the opening 18 may have a length of between about 0.5 and 1.0 inches and a width of between about 1.75 and 2.25 inches, and the body 12 may have a length of between about 2.5 to 4.0 inches and a width of between about 4.25 and 6.0 inches. It is understood, however, that these embodiments are not intended to be limiting, and the liner 10 could be configured to fit any size or shape of CPAP mask M.

Turning to FIG. 4, a forehead liner 24 may also be provided to interface with a forehead stabilizer portion S of a respiratory mask M (see FIG. 5) to create a two-piece liner system in accordance with an embodiment. The forehead liner 24 is configured to be releasably held by the forehead stabilizer portion S and the user's face, and may have an area at least as large as an area of the forehead stabilizer portion S. The forehead liner 24 may have a generally rectangular shape and may comprise a single or multi-layer material such as, but not limited to, cotton. In one embodiment, the forehead liner 24 comprises three layers of an absorbent material. The forehead liner 24 may have a shape that is generally similar to the shape of the forehead stabilizer portion S and may extend beyond the perimeter of the forehead stabilizer portion S by between about 0.5 to 0.75 inches, although it is understood that the forehead liner 24 is not limited to this configuration. Liner 10 and forehead liner 24 may be used together but may also be used separately as desired by a user.

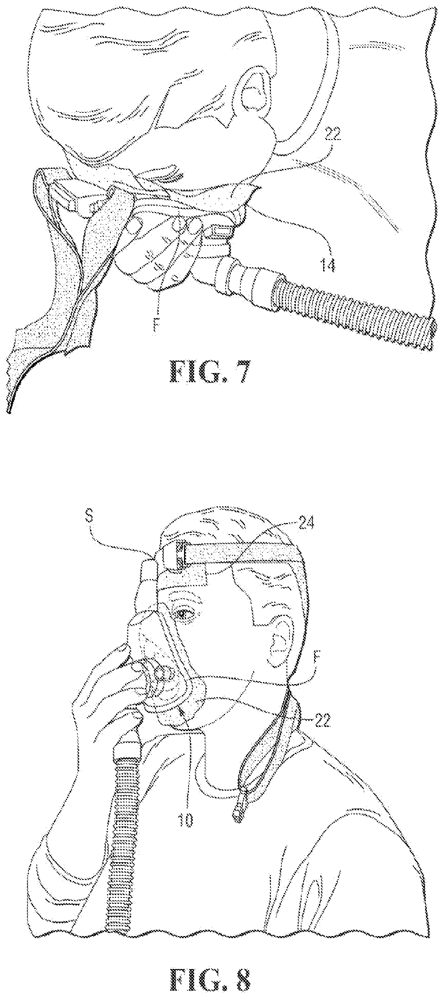

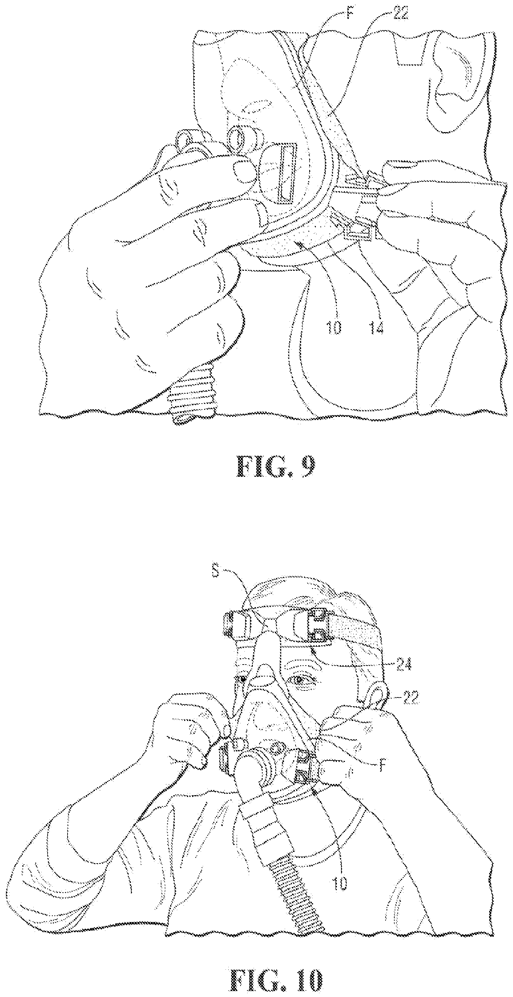

Referring now to FIGS. 6-10, a method of using the liner 10 according to an embodiment will be described. As shown in FIG. 6, the liner 10 may be placed over the face-engaging portion F such that the outer edge 14 extends beyond and is spaced apart from the face-engaging portion F. Although not shown in this figure, the forehead liner 24 can also be placed on the forehead stabilizer portion S if desired. The user may then lean his/her face downward toward the mask M, fitting his/her nose and/or mouth (as applicable) into the opening 18 as depicted in FIG. 7. Next, the user may press his/her face against the liner 10 and mask M while returning his/her head to a normal upright position as shown in FIG. 8. As illustrated in FIG. 9, the user may then snap the mask fasteners into place and adjust their tightness to secure the mask M. Securing the mask M releasably holds the liner 10 between the face-engaging portion F and a user's face, such that the liner 10 regulates air flow and reduces air leaks between the face-engaging portion F and the user's face. Lastly, with reference to FIG. 10, the liner 10 may be adjusted, such as around the nose and mouth, by pulling outward on the protruding extending portion 22, thereby providing a customized fit for a particular user. Of course, it is understood that variations on the above-described use of liner 10 and forehead liner 24 are fully contemplated.

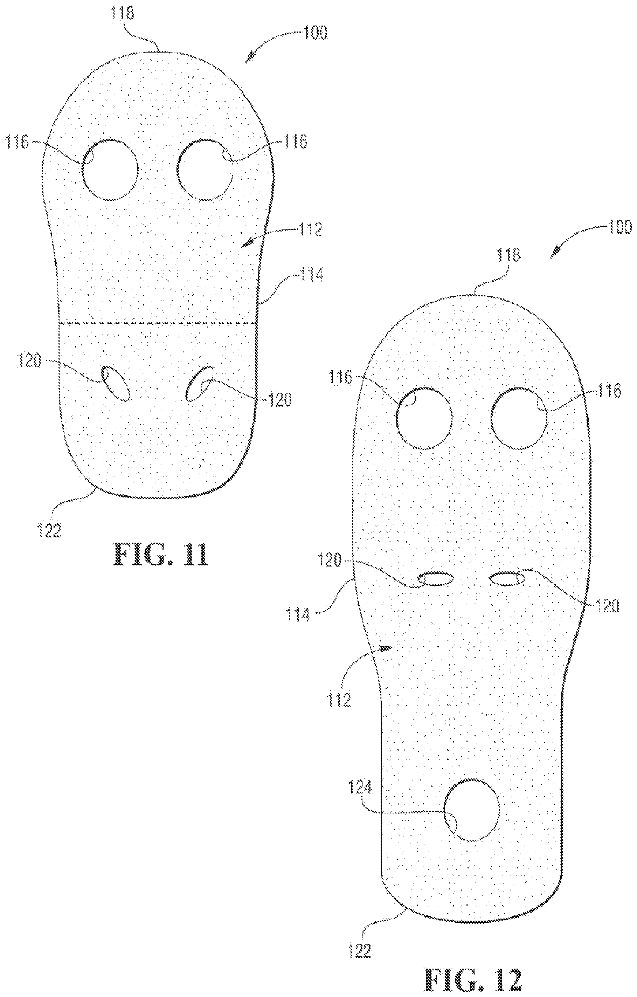

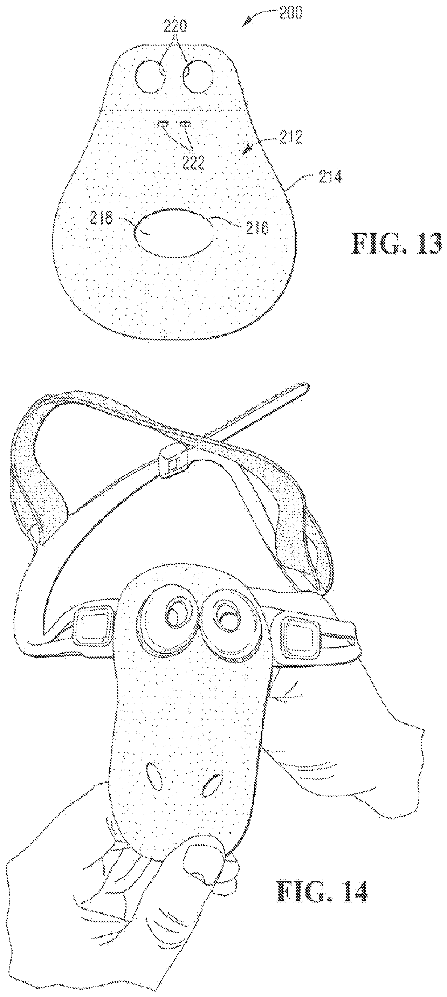

In another embodiment, a liner 100 as illustrated in FIG. 11 is provided which includes a plurality of apertures, such as for use with a nasal pillow mask, as shown in FIG. 14, for example. The liner 100 includes a body 112 having an outer edge 114, a first pair of apertures 116 which may be adjacent a first end 118 of the liner 100, and a second pair of apertures 120 which may be adjacent a second end 122 of the liner 100. The body 112 may be generally oval-shaped, elliptical, round, triangular, or rectangular, or have any other shape appropriate for use with a respiratory mask and is not limited to the shape depicted herein. The first pair of apertures 116 are sized to be placed over the nasal pillows of a nasal pillow mask for maintaining proper position of the liner 100 and, in a non-limiting example, may be approximately 0.5 to 1.0 inches in diameter. The second pair of apertures 120 are sized to lay on top of the nasal pillows to provide comfort to a user's nostrils and allow air to flow through the nasal pillows and into the user's nostrils. The second pair of apertures 120 may be generally circular or oval-shaped as shown but are not intended to be limited to these shapes.

In another embodiment, which may be for use with a nasal pillow mask, the liner 100 may include an additional aperture 124 sized for placement over the hose connection of the mask for possible added stability. In this embodiment, the second pair of apertures 120 may be disposed more centrally along the length of the liner 100.

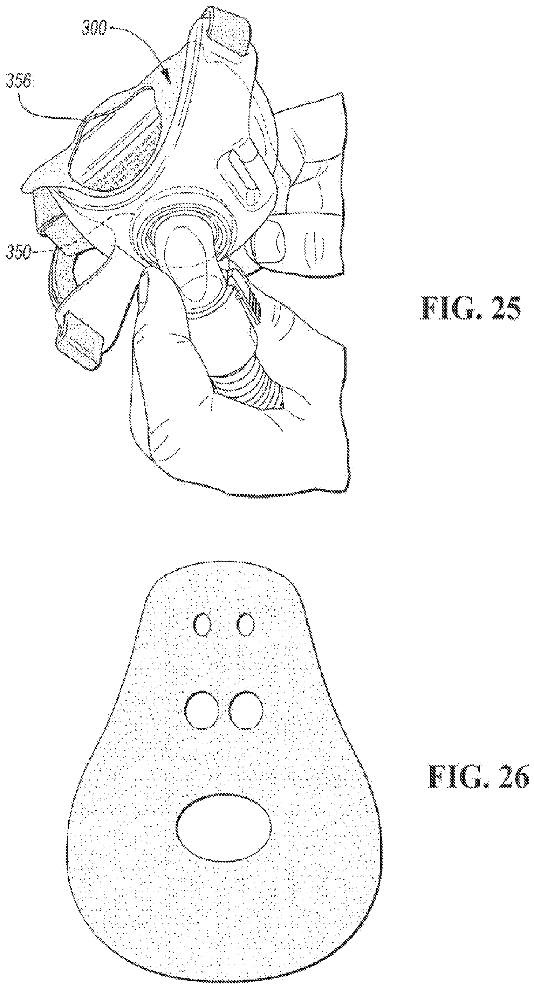

For a hybrid face-nasal pillow mask, the embodiment of FIG. 13 may be utilized. The liner 200 includes a body 212 having an outer edge 214, an inner edge 216, and an opening 218 bounded by the inner edge 216. The body 212 may be generally oval-shaped, elliptical, round, triangular, or rectangular, or have any other shape appropriate for use with a respiratory mask and is not limited to the shape depicted herein. The opening 218 is configured to at least partially receive the mouth, allowing air flow from an air source to be received by the user through the mask. The opening 218 may be generally elliptical or oval-shaped as shown but is not intended to be limited to these shapes. The liner 200 further includes a first pair of apertures 220, which may be adjacent an end of the liner 200, and a second pair of apertures 222 set inward from the first pair of apertures 220. The first pair of apertures 220 are sized to be placed over the nasal pillows for maintaining proper position of the liner 100 and, in a non-limiting example, may be approximately 0.5 to 1.0 inches in diameter. The second pair of apertures 222 are sized to lay on top of the nasal pillows to provide comfort for a user's nostrils and allow air to flow through the nasal pillows and into the user's nostrils. The second pair of apertures 222 may be generally circular or oval-shaped as shown but are not intended to be limited to these shapes. FIG. 26 illustrated another, related embodiment, wherein the description of FIG. 13 may also be applicable.

In the embodiment of FIG. 11, the body 112 may have a length of between about 4.0 to 5.0 inches and a width of between about 2.0 to 3.0 inches. In the embodiment of FIG. 12, the length of the body 112 may be between 6.0 and 7.0 inches and the width of the body may be between about 2.0 to 3.0 inches. In the embodiment of FIG. 13, the opening 218 may have a length of between about 1.0 to 3.0 inches and a width of between about 1.0 to 1.75 inches, and the body 212 may have a length of between about 6.0 to 7.0 inches. The body 212 may have a width of between about 5.0 to 6.0 inches at a bottom portion thereof adjacent the opening 218 and between about 2.5 to 3.5 inches at a top portion thereof adjacent the first pair of apertures 220. It is understood, however, that these embodiments are not intended to be limiting, and the liners 100, 200 could be configured to fit any size or shape of CPAP mask.

In use, the liners 100, 200 may be positioned between and held in place by the respiratory mask and the face of a user in order to absorb moisture, maintain proper positioning of the mask, and greatly reduce or eliminate air from leaking between the mask and the user's face. A perimeter of the liner outer edge 114, 214, which may be continuous or discontinuous, is larger than a perimeter of the face-engaging portion (including nasal pillows) of the mask, wherein the liners 100, 200 are configured to be releasably held between the mask and a user's face such that the outer edge 114, 214 extends beyond the mask face-engaging portion around at least part of its perimeter. As such, the body 112, 212 has an extending portion outward of the perimeter of the face-engaging portion. By allowing the outer edge 114, 214 of the liner 100, 200 to loosely protrude beyond the mask, the extending portion is configured to be in non-adhering communication with a user's face and serves to reduce air leaks from the perimeter of the mask by acting as a baffle to regulate, limit, or diffuse air flow between the mask and the skin, thus also stopping any resulting squealing-type noises created by such air leaks. It is understood that the other features described above with reference to liner 10 may also be applicable to liners 100, 200.

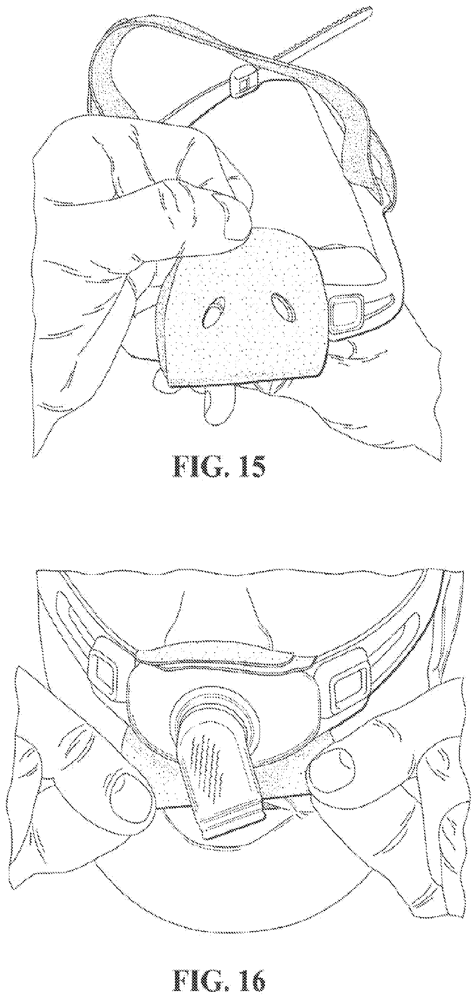

With reference to FIGS. 14-16, a method of using liner 100 according to an embodiment is described below. FIG. 14 illustrates placement of the first pair of apertures 116 over the nasal pillows of a nasal pillow mask. FIG. 15 illustrates a user folding the liner 100 so that the second pair of apertures 120 is aligned with the nasal pillows, wherein in one embodiment the fold may occur approximately at a location as indicated by dashed lines in FIG. 11. The user may then place his/her nostrils over the top of the nasal pillows with the liner 100 in between and proceed to secure the mask. FIG. 16 illustrates a user adjusting the positioning of the liner 100 if desired by pulling on the extending portion of the liner body 112 protruding beyond the perimeter of the nasal pillow mask.

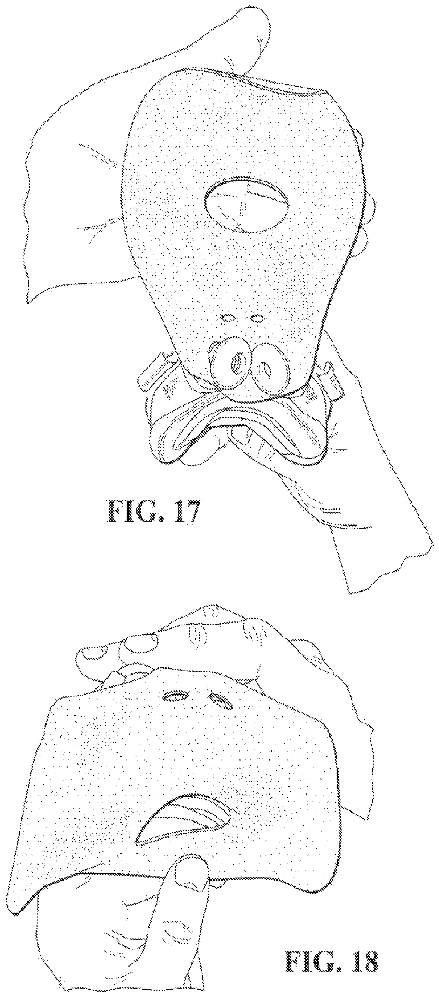

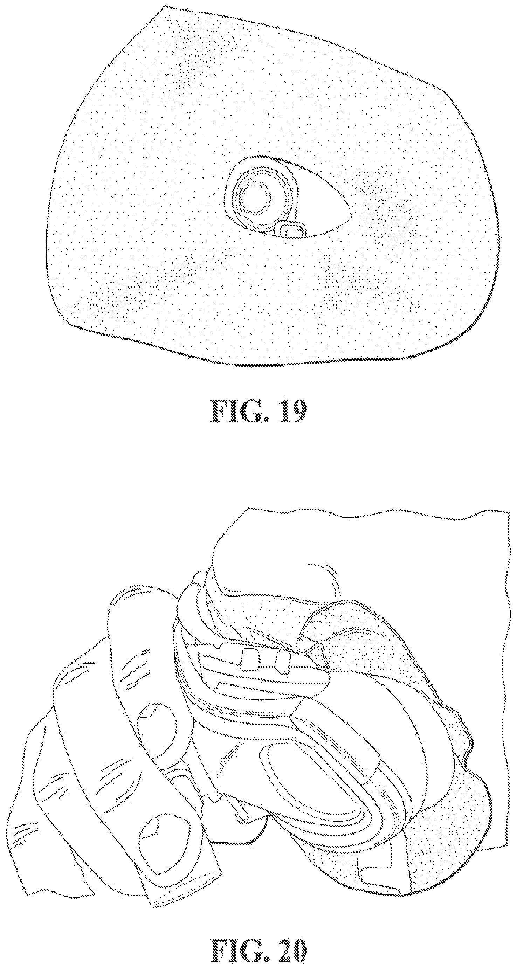

With reference to FIGS. 17-20, a method of using liner 200 according to an embodiment is described below. FIG. 17 illustrates placement of the first pair of apertures 220 over the nasal pillows of a hybrid nasal pillow/partial face mask. FIG. 18 illustrates a user folding the liner 200 so that the second pair of apertures 222 is aligned with the nasal pillows, wherein in one embodiment the fold may occur approximately at a location as indicated by dashed lines in FIG. 13. FIG. 19 illustrates alignment of the opening 218 with the mouthpiece and face-engaging portion of the hybrid mask. FIG. 20 illustrates a user engaging the liner 200 placed on the hybrid mask, fitting his nostrils to the nasal pillows and mouth into the liner opening 218, after which the user can proceed to secure the mask. The liner 200 may then be adjusted, such as around the nose and mouth, by pulling outward on the protruding extending portion, thereby providing a customized fit for a particular user. Of course, it is understood that variations on the above-described use of liners 100, 200 are fully contemplated.

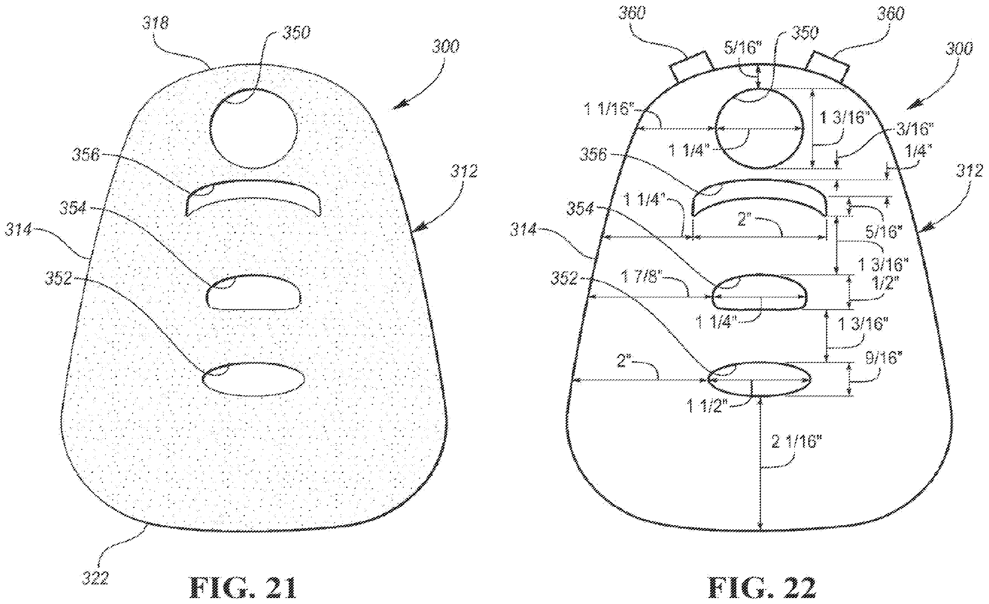

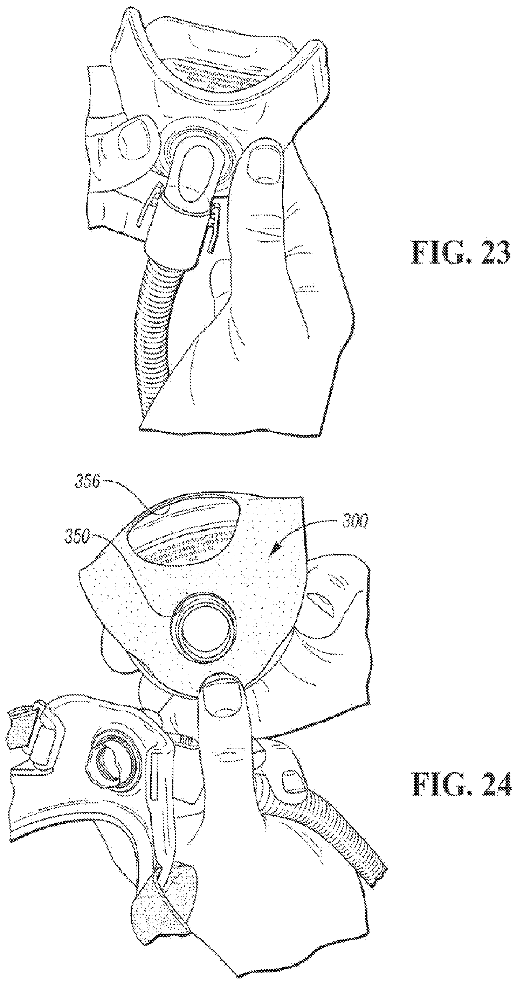

In another embodiment, a liner 300 as illustrated in FIG. 21 is provided which includes a plurality of apertures, such as for use with a respiratory mask wherein the frame portion is detachable from the cushion portion, as shown in FIG. 23, for example. The liner 300 includes a body 312 having an outer edge 314, and a hose aperture 350 which may be adjacent a first end 318 of the liner 300 and sized for placement over the hose connector of the mask. The liner 300 includes a mouth aperture 352 which may be adjacent a second end 322 of the liner 300 and configured for placement over the mouth opening of the mask to allow air to flow into the user's mouth, and a nasal aperture 354 set farther inward from the second end 322 compared to the mouth aperture 352 and configured for placement over the nasal opening of the mask to allow air to flow into the user's nostrils. In an alternative embodiment, it is contemplated that a combined mouth and nasal opening could be provided in the mask, such that mouth aperture 352 and nasal aperture 354 could be formed as a single aperture. In addition, the liner 300 may include an export aperture 356 disposed between the hose aperture 350 and the nasal aperture 354, wherein the export aperture 356 is configured for placement over an export air portion of the mask, such as the plurality of air holes illustrated in FIG. 23.

The body 312 may be generally oval-shaped, elliptical, round, triangular, or rectangular, or have any other shape appropriate for use with a respiratory mask and is not limited to the shape depicted herein. The apertures 350, 352, 354 and 356 may be generally circular or elongate or oval-shaped as shown but are not intended to be limited to these shapes. With reference to FIG. 22, exemplary dimensions for the configuration of liner 300 are shown. It is understood, however, that these dimensions are not intended to be limiting, and the liner 300 could be configured to fit any size or shape of CPAP mask.

In use, the liner 300 may be positioned between and held in place by the respiratory mask and the face of a user in order to absorb moisture, maintain proper positioning of the mask, and greatly reduce or eliminate air from leaking between the mask and the user's face. A perimeter of the liner outer edge 314, which may be continuous or discontinuous, is larger than a perimeter of the face-engaging portion of the mask, wherein the liner 300 is configured to be releasably held between the mask and a user's face such that the outer edge 314 extends beyond the mask face-engaging portion around at least part of its perimeter. As such, the body 312 has an extending portion outward of the perimeter of the face-engaging portion. By allowing the outer edge 314 of the liner 300 to loosely protrude beyond the mask, the extending portion is configured to be in non-adhering communication with a user's face and serves to reduce air leaks from the perimeter of the mask by acting as a baffle to regulate, limit, or diffuse air flow between the mask and the skin, thus also stopping any resulting squealing-type noises created by such air leaks. It is understood that the other features described above with reference to liners 10, 100 and 200 may also be applicable to liner 300.

With reference to FIGS. 24-25, a method of using liner 300 according to an embodiment is described below. FIG. 24 illustrates placement of the liner 300 with the frame detached such that the hose aperture 350 is received over the hose connector of the mask, the export aperture 356 is aligned with an export air portion of the mask, the nasal aperture 354 is aligned with a nasal opening of the mask, and the mouth aperture 352 is aligned with a mouth opening of the mask. The user may then align his/her nostrils with the nasal aperture 354 and his/her mouth with the mouth aperture 352 and proceed to secure the mask. FIG. 24 illustrates the frame attached to the mask with the liner 300 in place. A user may adjust the positioning of the liner 300 if desired by pulling outward on the extending portion of the liner body 312 protruding beyond the perimeter of the respiratory mask, thereby providing a customized fit for a particular user. In one embodiment (FIG. 22), at least one adhesive tab 360 may be provided at liner first end 318 for securing the liner 300 to the mask, such as near the hose connection, thus helping to ensure that export aperture 356 does not in any way obstruct the export air portion of the mask when the liner 300 is in use with the mask. Tabs 360 do not change the configuration of the outer edge 314 of the liner 300 in the area of the nasal and mouth openings of the mask, where the outer edge 314 still loosely protrudes beyond the mask as described above. Of course, it is understood that variations on the above-described use of liner 300 are fully contemplated.

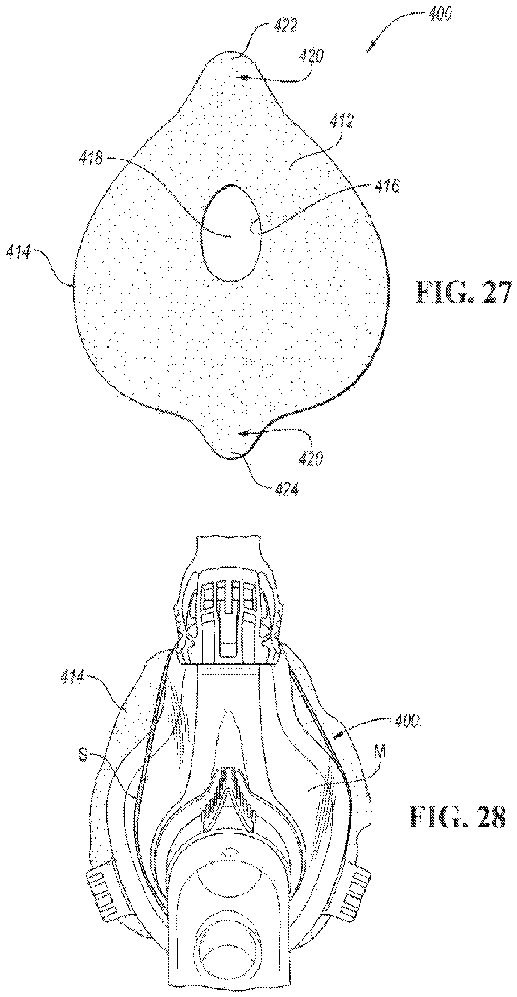

In some situations, it may be desirable to temporarily tether or secure at least part of the liner to the respiratory mask. For example, in hospital environments where respiratory masks may need to be removed or adjusted frequently by medical personnel during patient care, retaining the liner on the mask during these manipulations may be helpful. With reference to FIG. 27, a liner 400 is shown which may be utilized in such instances. The liner 400 includes a body 412 having an outer edge 414, an inner edge 416, and an opening 418 bounded by the inner edge 416. The body 412 may be generally oval-shaped, elliptical, round, triangular, or rectangular, or have any other shape appropriate for use with a respiratory mask and is not limited to the shape depicted herein. The opening 418 is configured to at least partially receive the mouth, allowing air flow from an air source to be received by the user through the mask. The opening 418 may be generally elliptical or oval-shaped as shown but is not intended to be limited to these shapes. The liner 400 may further include a flap 420 at one or both of a top end 422 and bottom end 424 of the liner 400 which may be used to tether the liner 400 to the respiratory mask M.

In one or more embodiments, the flaps 420 do not include adhesive or any other affixing material for securing the liner 400 to the mask M. Instead, the flaps 420 may be temporarily tethered to the respiratory mask by a strap S such as, for example, a rubber band. In one or more embodiments, each flap 420 may have a width approximately as wide as the opening 418. In one or more embodiments, each flap 420 may have a length that is between about 20% to 50% of the length from the opening 418 to the top end 422 or from the opening 418 to the bottom end 424. Such dimensions may help in providing adequate material to facilitate gripping of the flaps 420 by the strap S. Of course, other dimensions for the flaps 420 are also fully contemplated.

The flaps 420 may be curved to minimize unnecessary material and possible obstruction with respect to the mask. In one or more embodiments, the flaps 420 may be formed as a reverse curve departing from the curved trajectory of the outer edge 414 at one or both of the top 422 and bottom 424 ends of the liner 400. Of course, other shapes of the flaps 420 may alternatively be utilized.

In the embodiment of FIG. 27, the body 412 may have a length of about 7 ¾ inches, with a length of about 2 5/8 inches from the opening 418 to the top end 422 and a length of about 3 ½ inches from the opening 418 to the bottom end 424 of the liner 400. The liner 400 may have a width of about 6 ¼ inches, and the opening 418 may have a length of about 1 ¾ inches and a width of about 1 ¼ inches. It is understood, however, that these dimensions are not intended to be limiting, and the liner 400 could be configured to fit any size or shape of respiratory mask.

In use, the liner 400 may be positioned between and held in place by the respiratory mask M and the face of a user in order to absorb moisture, maintain proper positioning of the mask, and greatly reduce or eliminate air from leaking between the mask and the user's face. A perimeter of the liner outer edge 414, which may be continuous or discontinuous, is larger than a perimeter of the face-engaging portion of the mask, wherein the liner 400 is configured to be releasably held between the mask and a user's face such that the outer edge 414 extends beyond the mask face-engaging portion around at least part of its perimeter. As such, the body 412 has an extending portion outward of the perimeter of the face-engaging portion. By allowing the outer edge 414 of the liner 400 to loosely protrude beyond the mask, the extending portion is configured to be in non-adhering communication with a user's face and serves to reduce air leaks from the perimeter of the mask by acting as a baffle to regulate, limit, or diffuse air flow between the mask and the skin, thus also stopping any resulting squealing-type noises created by such air leaks. It is understood that the other features described above with reference to liners 10, 100, 200 and 300 may also be applicable to liner 400.

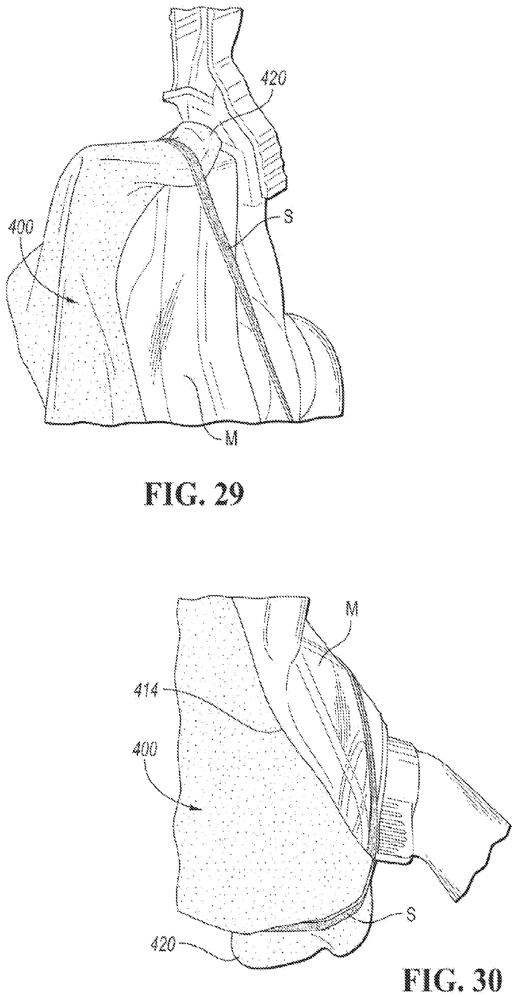

FIGS. 28-30 illustrate placement of the liner 400 on a respiratory mask M such that the opening 418 is aligned with a mouth opening of the mask, where a strap S is used to engage the flaps 420 and tether the liner 400 to the mask M. As shown, tethering the liner 400 to the mask via the flaps 420 does not change the configuration of the outer edge 414 of the liner 400 in the area of the mouth opening of the mask, where the outer edge 414 still loosely protrudes beyond the mask as described above. Of course, it is understood that variations on the above-described use of the liner 400 are fully contemplated.

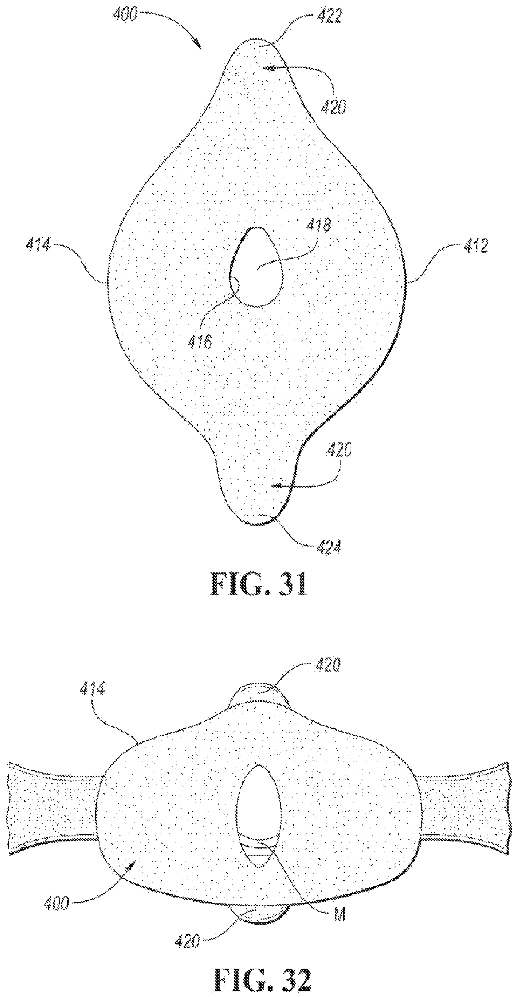

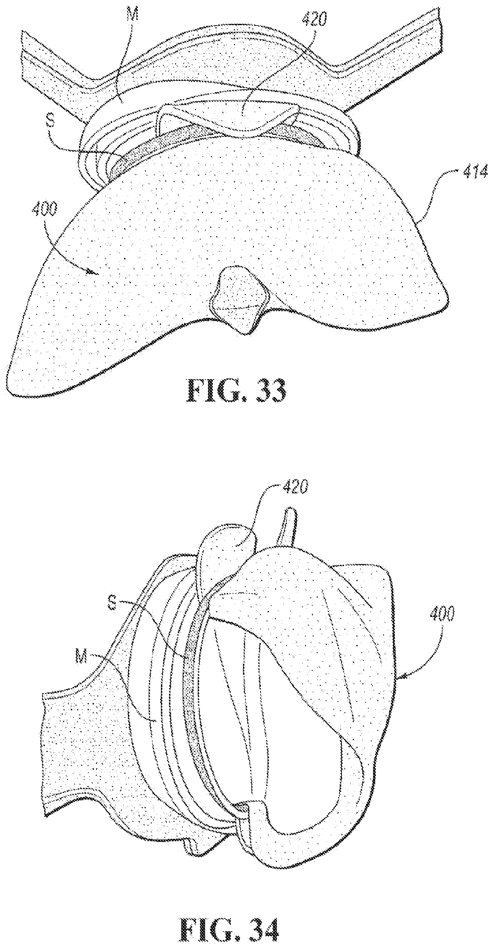

FIG. 31 depicts an embodiment of the liner 400 which may be suitable for use with a pediatric respiratory mask. In the embodiment of FIG. 31, the body 412 may have a length of about 3 5/8 inches, with a length of about 1 9/16 inches from the opening 418 to the top end 422 and a length of about 1 5/8 inches from the opening 418 to the bottom end 424 of the liner 400. The liner 400 may have a width of about 2 1/2 inches, and the opening 418 may have a length of about 9/16 inches and a width of about ½ inch. Again, it is understood that these dimensions are not intended to be limiting, and the liner 400 could be configured to fit any size or shape of CPAP mask. As above, FIGS. 32-34 illustrate placement of the liner 400 on a respiratory mask M such that the opening 418 is aligned with a mouth opening of the mask, where a strap S is used to engage the flaps 420 and tether the liner 400 to the mask M while allowing the liner outer edge 414 to loosely protrude beyond the mask.

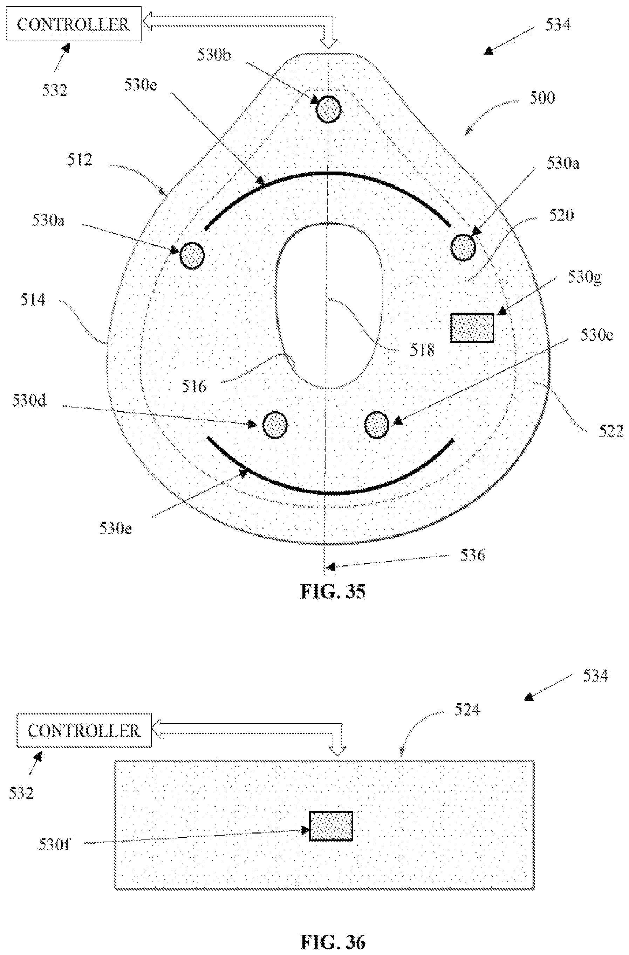

The liners 10, 100, 200, 300, 400 described above may also include "smart" functionality in the form of one or more sensors disposed on, attached to, or integrated within the material or textile of the body of the liner. Such a liner 500 is illustrated in FIG. 35. The liner 500 includes a body 512 having an outer edge 514, an inner edge 516, and an opening 518 bounded by the inner edge 516. The liner 500 may comprise a single or multi-layer material such as, but not limited to, cotton. The body 512 may be generally oval-shaped, elliptical, round, triangular, or rectangular, or have any other shape appropriate for use with a respiratory mask and is not limited to the shape depicted herein. The liner 500 and the opening 518 are sized and configured to at least partially receive the nose, mouth, or both nose and mouth, depending upon the type of mask, allowing air flow from an air source to be received by the user through mask. The opening 518 may be generally elliptical or oval-shaped as shown but is not intended to be limited to these shapes. The liner 500 could be configured to fit any size or shape of respiratory mask.

In use, the liner 500 may be positioned between and held in place by the respiratory mask and the face of a user in order to absorb moisture, maintain proper positioning of the mask, and greatly reduce or eliminate air from leaking between the mask and the user's face. A perimeter of the liner outer edge 514, which may be continuous or discontinuous, is larger than a perimeter of the face-engaging portion of the mask, wherein the liner 500 is configured to be releasably held between the mask and a user's face such that the outer edge 514 extends beyond the mask face-engaging portion around at least part of its perimeter. As such, the body 512 has an extending portion outward of the perimeter of the face-engaging portion. By allowing the outer edge 514 of the liner 500 to loosely protrude beyond the mask, the extending portion is configured to be in non-adhering communication with a user's face and serves to reduce air leaks from the perimeter of the mask by acting as a baffle to regulate, limit, or diffuse air flow between the mask and the skin, thus also stopping any resulting squealing-type noises created by such air leaks. It is understood that the other features described above with reference to liners 10, 100, 200, 300 and 400 may also be applicable to liner 500.

With reference to FIG. 36, a forehead liner 524 may also be provided to interface with a forehead stabilizer portion S of a respiratory mask M (see FIG. 5) to create a two-piece liner system in accordance with an embodiment. The forehead liner 524 is configured to be releasably held by the forehead stabilizer portion S and the user's face, and may have an area at least as large as an area of the forehead stabilizer portion S. The forehead liner 524 may have a generally rectangular shape and may comprise a single or multi-layer material such as, but not limited to, cotton. In one embodiment, the forehead liner 524 comprises three layers of an absorbent material. The forehead liner 524 may have a shape that is generally similar to the shape of the forehead stabilizer portion S and may extend beyond the perimeter of the forehead stabilizer portion S by between about 0.5 to 0.75 inches, although it is understood that the forehead liner 524 is not limited to this configuration. Liner 500 and forehead liner 524 may be used together but may also be used separately as desired by a user.

The liner 500 and/or the forehead liner 524 may further include at least one sensor 530. A controller 532 is provided in communication with the one or more sensors 530, wherein this system is designated with reference numeral 534 in FIGS. 35 and 36. The one or more sensors 530 can be affixed to one side of the liner 500, 524, such as an inner side, such as via adhesive. Alternatively, the one or more sensors 530 could be attached to the liner 500, 524, such as via fasteners or a hook-and-loop material (e.g., Velcro^{®}) so as to be removable and repositionable on the liner 500, 524. If the sensor 530 does not require direct contact with the skin of the user, the sensor 530 could be disposed within the textile material of the liner 500, 524 so as to be partially or completely covered by the liner material.

Various types of sensors 530 are contemplated in connection with the liner 500 and/or forehead liner 524. For example, an electrodermal sensor 530a, such as a galvanic skin response sensor, could be used to monitor perspiration levels or electrodermal conductivity of the skin of the user. In the non-limiting example shown in FIG. 35, two electrodermal sensors 530a are utilized, one positioned on each side (e.g., left and right) of the body 512 of the liner 500.

In another example, a gyroscope and/or accelerometer sensor 530b may be utilized to determine the position in space of the liner 500, 524, and thus the user, and thereby monitor movement. The gyroscope may provide 3D-axis positioning of the user, while the accelerometer may detect motion change forces (and may operate in tandem with the gyroscope for improved 3D space position modeling). In the non-limiting example shown in FIG. 35, the accelerometer 530b may be positioned near one end of the body 512 (e.g., the top end), such as along a longitudinal axis 536 of the liner 500.

In another example, a pulse oximeter (SpO₂) sensor 530c may be used to detect blood oxygen levels (oxygen saturation) and heart rate of the user, and may be positioned on the body 512 of the liner 500. The sensor 530c could additionally or alternatively include an EKG monitor for continuous monitoring of a user's pulse as well as EKG pattern recognition. A temperature sensor 530d for monitoring surface skin temperature of the user could also be provided on the liner 500.

With continuing reference to FIG. 35, the liner 500 may also include one or more pressure sensors 530e, such as in the form of pressure-sensitive materials which change color on a gradient. For example, a pressure sensitive material 530e, such as a strip (not limited to the length or shape depicted herein), may be disposed on, attached to, or integrated within the body 512 of the liner 500 at one or more locations. In one exemplary embodiment, the pressure sensor 530e may show dark red in areas with the most pressure on the liner 500 (and user) and lighter pink in areas with less pressure. The pressure sensitive material 530e may be calibrated to show particular color responses based on predetermined levels of pressure so that accurate measurements can be obtained. This output may provide a general map of pressure gradient across the liner 500, and thus the user's face, for monitoring of pressure "hotspots".

While described above with reference to liner 500, it is understood that sensors 530a, 530b, 530c, 530d and/or 530e could alternatively be disposed on, attached to, or integrated within the forehead liner 524. With reference to FIG. 36, a noninvasive EEG sensor 530f may be positioned on the forehead liner 524 for monitoring brain wave activity and patterns of the user. In addition to the above sensors 530, a pH sensor 530g, such as a litmus test strip material, may be disposed on, attached to, or integrated into the liner 500, 524 in order to monitor (e.g., via color change) possible pH fluctuations, bacterial colonies, and the presence of blood, plasma, or other predetermined substances on the user's skin.

It is understood that the sensors 530 are not limited to the types, placement, and configurations shown and described herein, and that other types, placements and configurations of sensors 530 are also fully contemplated.

As shown in FIGS. 35-36, the controller 532 may have one- or two-way communication with the at least one sensor 530 on the liner 500, 524, such as using a wireless communication protocol including, but not limited to, Bluetooth, WiFi, cellular networks, or another suitable protocol. The sensors 530 may also be interconnected so as to communicate with each other in a wired or wireless manner.

The controller 532 receives signals from the sensors 530 with data for further analysis. In one or more embodiments, the controller 532 may comprise a smartphone, computer, mobile device, or other suitable device, and may function to aggregate and process relevant information from a periodic or continuous stream of incoming data from the sensors 530. In other embodiments, a wearable device, such as an anklet or bracelet, could be employed in wireless communication with the sensors 530 and/or the controller 532.

The controller 532, in turn, may communicate the data from the sensors 530 to a physician or a healthcare network, for example, for monitoring patient care. The sensors 530 enable a healthcare practitioner to gain valuable insight into the variables that comprise a successful deployment of CPAP-related equipment for a particular user. Many times, the practitioner blindly adjusts strap tension and other settings hoping to achieve a comfortable fit for the user, only to be met with failure, mostly related to comfort issues. This process is frequently repeated over and over as various products are evaluated outside of a quantitative framework with real data acting as the guide. The system 534 disclosed herein may provide an equipment-agnostic framework of data which will enable a practitioner to pinpoint areas of failure in equipment deployment. This should enable a better, more efficient pairing of the right equipment for each individual user rather than locking a user into a particular brand, and provide actionable information related to true patient comfort.

The sensors 530 enable collection of data from which real-world conclusions can be drawn and presented to users. The controller 532 may include algorithms which produce relevant suggestions based on the gathered and analyzed data from the sensors 530, such as changes in sleep hygiene or bedtime routines, in addition to predictive analytics which can provide the user with a forecast or forward-looking information relative to their current environment. An example of the latter type of suggestion may include a prompt for the user to spend less time exposed to blue light from various electronic devices close to bedtime (e.g., based on the analysis of biometric data points). In addition, the controller 532 may use the sensor data to predict that a particular user is at risk of developing sleep or medical issues based on some identifiable trend (such as based on biometric data points, for example, EEG and/or electrodermal and heart rate data). Advantageously, the system 534 disclosed herein provides the ability to offer real, usable advice to users based on periodic or continuous data collection from the sensors 530 associated with the liner 500, 524.

Copper is a natural mineral having human nutritional benefit. Copper is also known to provide antimicrobial and potential wound healing properties. In one embodiment, the liners 10, 100, 200, 300, 400, 500 described herein may be manufactured with a copper or copper oxide material, such as CUPRON^{®}.

CPAP therapy is the most widely used method for treating sleep apnea, but it is only successful when the equipment fits and works properly and allows the user to stay asleep and experience deep-sleep, rapid eye movement (REM) cycles, the sleep cycle in which the most beneficial rest takes place. The liners 10, 100, 200, 300, 400, 500 may facilitate a more comfortable and effective CPAP therapy by contributing to a good fit of the CPAP mask M, providing comfort to the user, and reducing or eliminating air leaks, thus resulting in less disruption of sleep.

While exemplary embodiments are described above, it is not intended that these embodiments describe all possible forms of the technology. Rather, the words used in the specification are words of description rather than limitation, and it is understood that various changes may be made without departing from the scope of the disclosure. Additionally, the features of various implementing embodiments may be combined to form further embodiments of the technology. The scope of the present invention is defined by the claims that follow.

## Claims

1. A liner (500) for use with a respiratory mask having a face-engaging portion (F), the liner (500) comprising:
a body (512) constructed from an absorbent material, the body (512) having an outer edge (514) defining a perimeter of the liner (500), an inner edge (516), and an opening (518) bounded by the inner edge (516), the body (512) configured to be in non-adhering communication with a face of a user, the liner (500) configured to be releasably held by the respiratory mask and the face,
**characterized by** at least one sensor (530) coupled to the body (512) and configured to monitor at least one physiological parameter of the user during use.

2. The liner (500) of claim **1,** wherein the perimeter of the liner (500) is larger than a perimeter of the face-engaging portion (F) of the respiratory mask for forming an extending portion of the body, wherein the extending portion is a baffle for regulating a flow of air away from the face-engaging portion.

3. The liner (500) of claim 1, wherein the liner (500) is devoid of affixing material for securing the liner (500) to the respiratory mask.

4. The liner (500) of claim 1, wherein the at least one sensor (530) is removably attached to the body (512) and configured to be repositionable on the body (512).

5. The liner (500) of claim 1, wherein the at least one sensor (530) is disposed within the body (512) so as to be at least partially covered by the absorbent material.

6. A liner system for use with a respiratory mask having a face-engaging portion (F) and a forehead stabilizer portion (S), the liner system comprising:
a liner (500) including a body (512) constructed from an absorbent material, the body (512) having an outer edge (514) defining a perimeter of the liner (500), an inner edge (516), and an opening (518) bounded by the inner edge (516), the body (512) configured to be in non-adhering communication with a face of a user, the liner (500) configured to be releasably held by the respiratory mask and the face; and
a forehead liner (524) having a generally rectangular shape and configured to be releasably held by the forehead stabilizer portion (S) and the face;
**characterized by** at least one sensor (530) coupled to one or more of the liner (500) or the forehead liner (524) and configured to monitor at least one physiological parameter of the user during use.

7. The liner (500) of claim 1 or the liner system of claim 6, wherein the at least one sensor includes an electrodermal sensor (530a) to monitor a perspiration level or electrodermal conductivity of skin of the user.

8. The liner of (500) claim 1 or the liner system of claim 6, wherein the at least one sensor includes a gyroscope or accelerometer sensor (530b) to monitor movement of the user.

9. The liner (500) of claim 1 or the liner system of claim 6, wherein the at least one sensor includes a pulse oximeter (SpO₂) sensor (530c) to detect at least one of oxygen saturation or heart rate of the user.

10. The liner (500) of claim 1 or the liner system of claim 6, wherein the at least one sensor (530) includes an EKG monitor for monitoring at least one of a pulse or EKG pattern of the user.

11. The liner (500) of claim 1 or the liner system of claim 6, wherein the at least one sensor includes a temperature sensor (530d) for monitoring skin temperature of the user.

12. The liner (500) of claim 1 or the liner system of claim 6, wherein the at least one sensor includes a pressure sensor (530e).

13. The liner (500) of claim 1 or the liner system of claim 6, wherein the at least one sensor includes a pH sensor (530g).

14. The liner system of claim 6, wherein the at least one sensor includes a noninvasive EEG sensor (530f) coupled to the forehead liner (524) for monitoring brain wave activity and patterns of the user.

15. A system for monitoring a characteristic of a user during use of a respiratory mask having a face-engaging portion (F), the system comprising:
a liner (500) according to claim 1 or the liner system of claim 6; and
a controller (532) in communication with the at least one sensor (530), the controller (532) configured to receive signals from the at least one sensor (530) with data relating to the at least one physiological parameter.

16. The system of claim 15, wherein the controller (532) is configured to communicate the data from the at least one sensor (530) to a physician or a healthcare network for monitoring user care.

17. The system of claim 15, wherein the at least one sensor (530) comprises a plurality of sensors that are configured to communicate with each other.

## Patentansprüche

1. Auskleidung (500) zur Verwendung mit einer Atemmaske, die einen Gesichtseingriff-Abschnitt (F) aufweist, wobei die Auskleidung (500) umfasst:
einen Körper (512), der aus einem absorbierenden Material aufgebaut ist, wobei der Körper (512) eine Außenkante (514), die einen Umfang der Auskleidung (500) definiert, eine Innenkante (516) und eine Öffnung (518), die durch die Innenkante (516) begrenzt wird, aufweist, wobei der Körper (512) dazu ausgelegt ist, in nicht haftender Verbindung mit einem Gesicht eines Benutzers zu stehen, wobei die Auskleidung (500) dazu ausgelegt ist, von der Atemmaske und dem Gesicht lösbar gehalten zu werden,
**gekennzeichnet durch** wenigstens einen Sensor (530), der an den Körper (512) gekoppelt ist und dazu ausgelegt ist, wenigstens einen physiologischen Parameter des Benutzers während der Verwendung zu überwachen.

2. Auskleidung (500) nach Anspruch 1, wobei der Umfang der Auskleidung (500) größer ist als ein Umfang des Gesichtseingriff-Abschnitts (F) der Atemmaske, um einen erweiterten Abschnitt des Körpers zu bilden, wobei der erweiterte Abschnitt eine Trennwand zum Regulieren eines Luftstroms weg von dem Gesichtseingriff-Abschnitt ist.

3. Auskleidung (500) nach Anspruch 1, wobei die Auskleidung (500) frei von Befestigungsmaterial zum Befestigen der Auskleidung (500) an der Atemmaske ist.

4. Auskleidung (500) nach Anspruch 1, wobei der wenigstens eine Sensor (530) entfernbar an dem Körper (512) angebracht und dazu ausgelegt ist, an dem Körper (512) repositionierbar zu sein.

5. Auskleidung (500) nach Anspruch 1, wobei der wenigstens eine Sensor (530) so innerhalb des Körpers (512) angeordnet ist, dass er wenigstens teilweise von dem absorbierenden Material bedeckt ist.

6. Auskleidungssystem zur Verwendung mit einer Atemmaske, die einen Gesichtseingriff-Abschnitt (F) und einen Stirnstabilisator-Abschnitt (S) aufweist, wobei das Auskleidungssystem umfasst:
eine Auskleidung (500), die einen Körper (512) aufweist, der aus einem absorbierenden Material aufgebaut ist, wobei der Körper (512) eine Außenkante (514), die einen Umfang der Auskleidung (500) definiert, eine Innenkante (516) und eine Öffnung (518), die durch die Innenkante (516) begrenzt wird, aufweist, wobei der Körper (512) dazu ausgelegt ist, in nicht haftender Verbindung mit einem Gesicht eines Benutzers zu stehen, wobei die Auskleidung (500) dazu ausgelegt ist, von der Atemmaske und dem Gesicht lösbar gehalten zu werden; und
eine Stirnauskleidung (524), die eine allgemein rechteckige Form aufweist und dazu ausgelegt ist, von dem Stirnstabilisator-Abschnitt (S) und dem Gesicht lösbar gehalten zu werden;
**gekennzeichnet durch** wenigstens einen Sensor (530), der an eines oder mehrere von der Auskleidung (500) oder der Stirnauskleidung (524) gekoppelt ist und dazu ausgelegt ist, wenigstens einen physiologischen Parameter des Benutzers während der Verwendung zu überwachen.

7. Auskleidung (500) nach Anspruch 1 oder Auskleidungssystem nach Anspruch 6, wobei der wenigstens eine Sensor einen elektrodermalen Sensor (530a) zum Überwachen eines Perspirationsniveaus oder einer elektrodermalen Leitfähigkeit der Haut des Benutzers umfasst.

8. Auskleidung nach (500) Anspruch 1 oder Auskleidungssystem nach Anspruch 6, wobei der wenigstens eine Sensor einen Gyroskop- oder Beschleunigungsmessersensor (530b) zum Überwachen von Bewegung des Benutzers umfasst.

9. Auskleidung (500) nach Anspruch 1 oder Auskleidungssystem nach Anspruch 6, wobei der wenigstens eine Sensor einen Pulsoximeter(SpO₂)-Sensor (530c) umfasst, um wenigstens eines von Sauerstoffsättigung oder Herzfrequenz des Benutzers zu erfassen.

10. Auskleidung (500) nach Anspruch 1 oder Auskleidungssystem nach Anspruch 6, wobei der wenigstens eine Sensor (530) einen EKG-Monitor zum Überwachen von wenigstens einem von einem Puls und einem EKG-Musters des Benutzers umfasst.

11. Auskleidung (500) nach Anspruch 1 oder Auskleidungssystem nach Anspruch 6, wobei der wenigstens eine Sensor einen Temperatursensor (530d) zum Überwachen von Hauttemperatur des Benutzers umfasst.

12. Auskleidung (500) nach Anspruch 1 oder Auskleidungssystem nach Anspruch 6, wobei der wenigstens eine Sensor einen Drucksensor (530e) umfasst.

13. Auskleidung (500) nach Anspruch 1 oder Auskleidungssystem nach Anspruch 6, wobei der wenigstens eine Sensor einen pH-Sensor (530g) umfasst.

14. Auskleidungssystem nach Anspruch 6, wobei der wenigstens eine Sensor einen nichtinvasiven EEG-Sensor (530f) umfasst, der an die Stirnauskleidung (524) gekoppelt ist, um Gehirnwellenaktivität und -muster des Benutzers zu überwachen.

15. System zum Überwachen einer Charakteristik eines Benutzers während der Verwendung einer Atemmaske mit einem Gesichtseingriff-Abschnitt (F), wobei das System umfasst:
eine Auskleidung (500) nach Anspruch 1 oder das Auskleidungssystem nach Anspruch 6; und
eine Steuerung (532) in Kommunikation mit dem wenigstens einen Sensor (530), wobei die Steuerung (532) dazu ausgelegt ist, Signale von dem wenigstens einen Sensor (530) mit Daten zu empfangen, die sich auf den wenigstens einen physiologischen Parameter beziehen.

16. System nach Anspruch 15, wobei die Steuerung (532) dazu ausgelegt ist, die Daten von dem wenigstens einen Sensor (530) an einen Arzt oder ein Gesundheitsnetzwerk zum Überwachen von Benutzerversorgung zu kommunizieren.

17. System nach Anspruch 15, wobei der wenigstens eine Sensor (530) eine Mehrzahl von Sensoren umfasst, die dazu ausgelegt sind, miteinander zu kommunizieren.

## Revendications

1. Doublure (500) destinée à être utilisée avec un masque respiratoire présentant une partie en contact avec le visage (F), la doublure (500) comprenant :
un corps (512) construit en matériau absorbant, le corps (512) présentant un bord externe (514) définissant un périmètre de la doublure (500), un bord interne (516), et une ouverture (518) délimitée par le bord interne (516), le corps (512) étant configuré pour être en communication non adhérente avec le visage d'un utilisateur, la doublure (500) étant configurée pour être maintenue de manière amovible par le masque respiratoire et le visage,
**caractérisée par** au moins un capteur (530) couplé au corps (512) et configuré pour surveiller au moins un paramètre physiologique de l'utilisateur lors de l'utilisation.

2. Doublure (500) selon la revendication 1, dans laquelle le périmètre de la doublure (500) est plus grand qu'un périmètre de la partie en contact avec le visage (F) du masque respiratoire pour former une partie d'extension du corps, dans laquelle la partie d'extension est un déflecteur pour réguler un écoulement d'air à l'opposé de la partie en contact avec le visage.

3. Doublure (500) selon la revendication 1, la doublure (500) étant dépourvue de matériau de fixation pour assujettir la doublure (500) au masque respiratoire.

4. Doublure (500) selon la revendication 1, dans laquelle l'au moins un capteur (530) est fixé de manière amovible au corps (512) et configuré pour être repositionnable sur le corps (512).

5. Doublure (500) selon la revendication 1, dans laquelle l'au moins un capteur (530) est disposé à l'intérieur du corps (512) de manière à être au moins partiellement recouvert par le matériau absorbant.

6. Système de doublure destiné à être utilisé avec un masque respiratoire présentant une partie en contact avec le visage (F) et une partie de stabilisation frontale (S), le système de doublure comprenant :
une doublure (500) incluant un corps (512) construit en matériau absorbant, le corps (512) présentant un bord externe (514) définissant un périmètre de la doublure (500), un bord interne (516), et une ouverture (518) délimitée par le bord interne (516), le corps (512) étant configuré pour être en communication non adhérente avec le visage d'un utilisateur, la doublure (500) étant configurée pour être maintenue de manière amovible par le masque respiratoire et le visage ; et
une doublure frontale (524) ayant une forme généralement rectangulaire et configurée pour être maintenue de manière libérable par la partie de stabilisation frontale (S) et le visage ;
**caractérisée par** au moins un capteur (530) couplé à la doublure (500) et/ou à la doublure frontale (524) et configuré pour surveiller au moins un paramètre physiologique de l'utilisateur lors de l'utilisation.

7. Doublure (500) selon la revendication 1 ou système de doublure selon la revendication 6, dans lesquels l'au moins un capteur comporte un capteur électrodermique (530a) pour surveiller un niveau de transpiration ou une conductivité électrodermique de la peau de l'utilisateur.

8. Doublure (500) selon la revendication 1 ou système de doublure selon la revendication 6, dans lesquels l'au moins un capteur comporte un gyroscope ou un capteur d'accéléromètre (530b) pour surveiller le mouvement de l'utilisateur.

9. Doublure (500) selon la revendication 1 ou système de doublure selon la revendication 6, dans lesquels l'au moins un capteur comporte un capteur d'oxymètre de pouls (SpO₂) (530c) pour détecter la saturation en oxygène et/ou la fréquence cardiaque de l'utilisateur.

10. Doublure (500) selon la revendication 1 ou système de doublure selon la revendication 6, dans lesquels l'au moins un capteur (530) comporte un moniteur EKG pour surveiller un pouls et/ou un motif EKG de l'utilisateur.

11. Doublure (500) selon la revendication 1 ou système de doublure selon la revendication 6, dans lesquels l'au moins un capteur comporte un capteur de température (530d) pour surveiller la température cutanée de l'utilisateur.

12. Doublure (500) selon la revendication 1 ou système de doublure selon la revendication 6, dans lesquels l'au moins un capteur comporte un capteur de pression (530e).

13. Doublure (500) selon la revendication 1 ou système de doublure selon la revendication 6, dans lesquels l'au moins un capteur comporte un capteur de pH (530g).

14. Système de doublure selon la revendication 6, dans lequel l'au moins un capteur comporte un capteur EEG non invasif (530f) couplé à la doublure frontale (524) pour surveiller l'activité et les motifs des ondes cérébrales de l'utilisateur.

15. Système permettant de surveiller une caractéristique d'un utilisateur lors de l'utilisation d'un masque respiratoire présentant une partie en contact avec le visage (F), le système comprenant :
une doublure (500) selon la revendication 1 ou le système de doublure selon la revendication 6 ; et
un dispositif de commande (532) en communication avec l'au moins un capteur (530), le dispositif de commande (532) étant configuré pour recevoir des signaux provenant de l'au moins un capteur (530) avec des données relatives à l'au moins un paramètre physiologique.

16. Système selon la revendication 15, dans lequel le dispositif de commande (532) est configuré pour communiquer les données provenant de l'au moins un capteur (530) à un médecin ou à un réseau de soins de santé pour surveiller les soins aux utilisateurs.

17. Système selon la revendication 15, dans lequel l'au moins un capteur (530) comprend une pluralité de capteurs qui sont configurés pour communiquer les uns avec les autres.
